# EUROPÄISCHE PATENTANMELDUNG

(11) **EP 3 269 718 A1**
(43) Veröffentlichungstag der Anmeldung: **17.01.2018**
(21) Anmeldenummer: 16178957.3
(22) Anmeldetag: 12.07.2016
(51) Int. Cl.: C07D 477/08, C07D 477/20, A61K 31/407, A61K 31/04

(54) **VERFAHREN ZUR ENTSCHÜTZUNG EINES CARBAPENEMS DURCH HETEROGENKATALYTISCHE HYDRIERUNG MIT WASSERSTOFF IN GEGENWART EINES ORGANISCHEN AMINS**

(71) Anmelder: Evonik Degussa GmbH, 45128 Essen (DE)
(72) Erfinder: GRÜNEWALD, Elena, 60388 Frankfurt (DE); WEIDLICH, Stephan, 63457 Hanau (DE); JANTKE, Ralf, 63776 Mömbris (DE)

(57) **Zusammenfassung**

Verfahren zur Herstellung eines Carbapenems oder dessen Vorstufe (I) durch Abspaltung einer oder mehrerer Schutzgruppen einer Substanz (II). 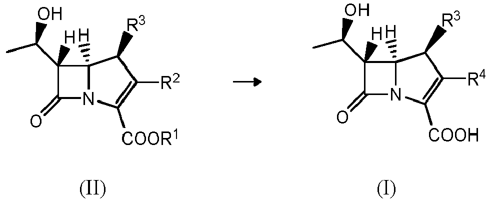 enthaltend die Schritte:
a) Hydrierung mit Wasserstoff eines Reaktionsgemisches (1) enthaltend die Substanz (II), mindestens ein organisches Lösungsmittel und mindestens ein aromatisches Amin in Gegenwart eines heterogenen Hydrierkatalysators unter Bildung eines Produktgemisches (1),
b) Abtrennung des heterogenen Katalysators aus dem Produktgemisch (1) unter Bildung eines Produktgemisches (2),
c) Hinzufügen von Wasser zum Produktgemisch (2), wobei das Gewichtsverhältnis von zugegebenem Wasser zum Produktgemisch (2) von 0,1 bis 10 ist,
d) Thermische Behandlung des Produktgemisches (II) unter Bildung eines Produktgemisches (3) enthaltend Substanz (I).

## Beschreibung

Die vorliegende Erfindung betrifft ein Verfahren zur Herstellung eines Carbapenems.

Carbapeneme sind β-Lactam-Antibiotika, die aufgrund ihres breiten antimikrobiellen Wirkspektrums als Arzneistoffe verwendet werden. Vertreter dieser Substanzklasse sind zum Beispiel Meropenem, Imipenem, Ertapenem, Doripenem, Tebipenem, Biapenem und Panipenem.

Es ist eine Vielzahl von Herstellungsverfahren für diese Verbindungen bekannt, wobei die Synthese dieser Verbindungen üblicherweise über mehrstufige Verfahren erfolgt. Ein wichtiger Schritt bei vielen Synthesen ist dabei die Entfernung von Schutzgruppen, auch Entschützung genannt. Die wichtigen Vertreter von Schutzgruppen in diesem Fall sind unsubstituierte und substituierte Benzyl- beziehungsweise Benzyloxycarbonylgruppen, wie zum Beispiel p-Nitrobenzyl (PNB) und p-Nitrobenzyloxycarbonyl (PNZ = CO₂PNB). Zur Herstellung der Carbapeneme sind aus dem Stand der Technik verschiedene Verfahren zur Entfernung der Schutzgruppen bekannt. Nachfolgend wird ein allgemeines Reaktionsschema zur Herstellung von Meropenem (**Ia**) durch Entfernung von PNZ- und PNB-Schutzgruppen aus dem geschützten Meropenem-Derivat (**IIa**) gezeigt:

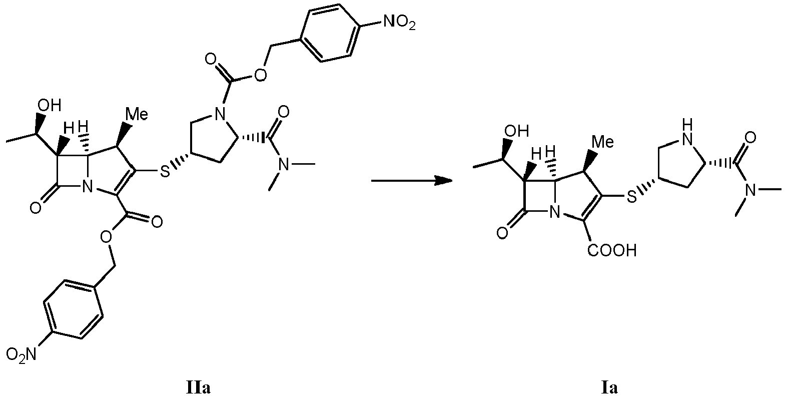

US4933333 offenbart ein Verfahren zur Herstellung von β-Lactam-Verbindungen. Es wird unter anderem ein Verfahren zur reduktiven Entfernung von substituierten Benzyl- und Benzyloxycarbonyl-Schutzgruppen, p-Nitrobenzyl beziehungsweise p-Nitrobenzyloxycarbonyl, beschrieben. In Beispiel 2 wird ein geschütztes Meropenemderivat in einem Gemisch aus THF und Ethanol gelöst und Morpholinopropansulfonsäure-Puffer und Hydrierkatalysator hinzugegeben. Die Reaktionsmischung wird hydriert, der Katalysator wird abfiltriert und THF und Ethanol werden abdestilliert. Der Rückstand wird mit Ethylacetat gewaschen, das Lösungsmittel erneut abdestilliert. Die wässrige Lösung wird mittels Polymer-Chromatographie gereinigt, um das entschützte Produkt, Meropenem, zu erhalten. Aus den Angaben lässt sich eine sehr geringe Konzentration des geschützten Meropenemderivats berechnen. Sie beträgt weniger als 1 Gew.-% in einer Mischung aus THF/Ethanol/Pufferlösung. Somit wird bei dieser Umsetzung eine erhebliche Menge an Lösungsmittel pro Mol Produkt benötigt, in Beispiel 2 > 60 mL Lösungsmittel / mmol Produkt. Dies hat einen direkten Einfluss auf die Produktionskosten.

CN102336756 offenbart ein Verfahren zur Herstellung von Meropenem durch reduktive Entschützung in einer einstufigen Reaktion. Die Reaktion findet in einem refluxierenden organischen Lösungsmittel in Gegenwart eines Hydrierkatalysators bei Normaldruck statt, wobei zusätzlich Wasser zugegeben werden kann. Als Katalysatoren können Pd, Pd/C, Palladiumhydroxid, oder Raney-Nickel eingesetzt werden. Als Reduktionsmittel werden Ameisensäure oder Ammoniumformiat eingesetzt. Als organische Lösungsmittel eignen sich Alkohole, Ether oder Ester. Es wird eine Ausbeute von ca. 50 % erhalten, die Konzentration an geschütztem Meropenem beträgt in Beispiel 1 ca. 3,5 Gew.-% in THF/Wasser, in Beispiel 2 ca. 3,7 Gew.-% in Methanol/Wasser. Bei der Umsetzung wird kein Wasserstoff als Reduktionsmittel eingesetzt. Auch in diesem Fall wird eine erhebliche Menge an Lösungsmittel pro Mol Produkt benötigt.

CN103450203 offenbart ein Verfahren zur Herstellung von Meropenem durch Eintopfsynthese eines geschützten Meropenemderivats und nachfolgende reduktive Entfernung einer PNB-Schutzgruppe. Als Lösungsmittel wird Tetrahydrofuran und/oder N,N-Dimethylformamid verwendet. Die Reduktion erfolgt mit Wasserstoff und Pd/C als Katalysator. Nach der Abtrennung des Katalysators erfolgt die Kristallisation von Meropenem durch Zugabe von Aceton oder Tetrahydrofuran. In den Beispielen 1-5 wird eine Ausbeute zwischen 69 % und 78 Mol % erhalten.

WO2007/104221 offenbart ein Verfahren zur Herstellung von Meropenem, bei dem die reduktive Entfernung einer PNB und einer PNZ-Schutzgruppe in einer einstufigen Reaktion in einem organischen-wässrigen Lösungsmittel in Gegenwart einer Base und eines Katalysators, Pd/C, bei einer Temperatur im Bereich von 0-100 °C stattfindet. Es wird eine Ausbeute von 65 % in Beispiel 2 beziehungsweise 71,8 % in Beispiel 3 erreicht. Die Konzentration des Einsatzstoffes lässt sich in Beispiel 2 zu ca. 3,0 Gew.-%, in Beispiel 3 zu ca. 2,4 Gew.-% berechnen. Die molare Menge des als Base eingesetzten 2,6-Dimethylpyridins bezogen auf die molare Menge des erhaltenen Produkts lässt sich für Beispiele 2 beziehungsweise 3 als 4,4 mol/mol in Beispiel 2 beziehungsweise 5,0 mol/mol in Beispiel 3 berechnen. Durch die Reaktionsführung mit einem THF-Wasser-Gemisch kann nur eine sehr geringe Eduktkonzentration eingesetzt werden. Dadurch ergibt sich ein erheblicher Bedarf an organischen Lösungsmitteln pro Gramm Einsatzstoff (> 90 mL/mmol Meropenem in Beispielen 2 und 3). Dies hat einen direkten Einfluss auf die Produktionskosten.

Die Aufgabe der vorliegenden Erfindung ist daher die Bereitstellung eines einfach durchführbaren wirtschaftlichen Entschützungsverfahrens zur Herstellung eines Carbapenems, bei dem unter anderem eine minimale Menge von Einsatzstoffe zum Einsatz kommen würde.

Es wurde nun gefunden, dass die Herstellung der Carbapeneme durch eine zweistufige Reaktionsführung beim Entschützen deutlich wirtschaftlicher als die im Stand der Technik beschriebenen Verfahren ist.

Alle Verfahren zur Herstellung von Carbapeneme durch Schutzgruppenentfernung aus dem Stand der Technik beschreiben eine einstufige Hydrierungs-/Entschützungsreaktion, wobei eine Lösung von geschütztem Carbapenem hydriert und direkt danach aufgearbeitet wird. Diese typische Vorgehensweise weist einige Nachteile auf. Entschützungsverfahren mit festen heterogenen Hydrierkatalysatoren, bei denen dem Reaktionsgemisch vor der Reduktion relativ viel Wasser zugegeben wird, ist eine gängige Ausführungsform. Dabei ist einerseits die Löslichkeit des Einsatzstoffes in einem Wasser-Lösungsmittel-Gemisch meistens sehr begrenzt, so dass nur eine geringe Konzentration des Einsatzstoffes erreicht werden kann und als Folge viel Lösungsmittel pro Gewichtseinheit Produkt benötigt wird. Das kann die Herstellungskosten des Produkts erheblich erhöhen. Andererseits kann die unterschiedliche Löslichkeit der Einsatzstoffe, Produkte und Zwischenprodukte in einem Wasser-Lösungsmittel-Gemisch dazu führen, dass Teile des Produkts oder Zwischenprodukts bereits während der katalytischen Umsetzung ausfallen können. Dies erschwert die Abtrennung des Produkts vom festen Hydrierkatalysator und kann zu einem Ausbeuteverlust an Zielprodukt Carbapenem führen.

Bei dem erfindungsgemäßen Verfahren werden geringere Mengen von organischen Lösungsmitteln benötigt. Außerdem kann die Reaktion auf diese Weise in einem viel breiteren Konzentrationsbereich durchgeführt werden. So ist es z.B. möglich, die Konzentration des geschützten Einsatzstoffes erheblich zu erhöhen und gleichzeitig Umsatz und Ausbeute nicht negativ zu beeinflussen.

Der Gegenstand der Erfindung ist ein Verfahren zur Herstellung einer Substanz der Formel (I) aus einer Substanz der Formel (II): 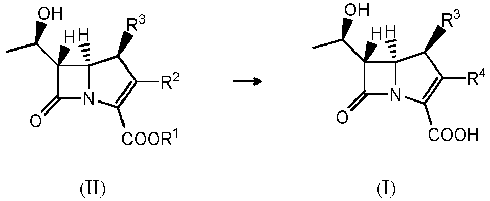 R¹ = p-Nitrobenzyl 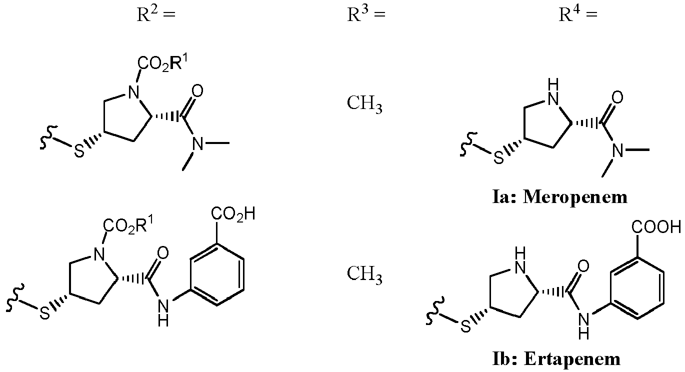 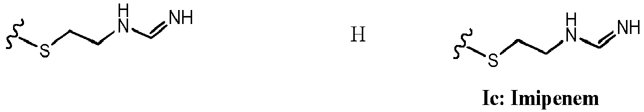 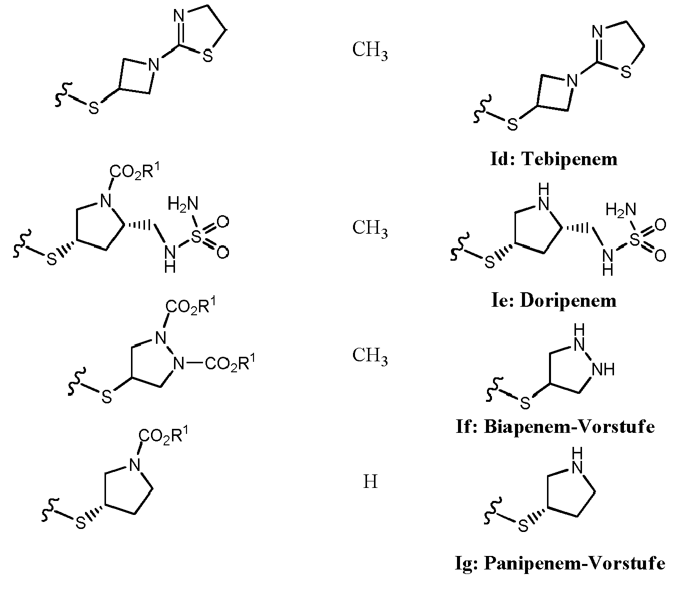 enthaltend die Schritte:
a) Hydrierung mit Wasserstoff eines Reaktionsgemisches (1) enthaltend Substanz (II), mindestens ein organisches Lösungsmittel und mindestens ein aromatisches Amin in Gegenwart eines heterogenen Hydrierkatalysators unter Bildung eines Produktgemisches (1),
b) Abtrennung des heterogenen Katalysators aus dem Produktgemisch (1) unter Bildung eines Produktgemisches (2),
c) Hinzufügen von Wasser zum Produktgemisch (2), wobei das Gewichtsverhältnis von zugegebenem Wasser zum Produktgemisch (2) von 0,1 bis 10 ist,
d) Thermische Behandlung des Produktgemisches (2) unter Bildung eines Produktgemisches (3) enthaltend Substanz (I).

Substanz (I) ist ein Carbapenem (**Ia-Ie**) oder dessen synthetische Vorstufe (**If**, **Ig**), Substanz (II) ist eine entsprechende mit p-Nitrobenzyl und/oder p-Nitrobenzyloxycarbonyl geschützte Carbapenemvorstufe.

Die Entschützung eines geschützten Carbapenems oder dessen Vorstufe, der Substanz (II), ist ein komplexer Vorgang, bei dem unter anderem die Löslichkeit des eingesetzten Eduktes und der gebildeten Zwischenprodukte sowie des Zielproduktes, Substanz (I), eine wichtige Rolle spielen. Die erste Phase des Prozesses, die katalytische Hydrierung einer im organischen Lösungsmittel gelösten Substanz (II) unter Bildung der primären Zwischenprodukte verläuft optimal, wenn kein beziehungsweise möglichst wenig Wasser dem Reaktionsgemisch (1) zugegeben wird und die Bestandteile des Produktgemisches (1) bis auf den eingesetzten heterogenen Katalysator gelöst bleiben. Es wurden Hinweise darauf gefunden, dass Wasser den Zerfall der während der katalytischen Hydrierung gebildeten Zwischenprodukte bis zum Zielprodukt, Substanz (I), fördert. Die Löslichkeit der Substanz (I) in einem organisch-wässrigen Lösungsmittelgemisch ist sehr begrenzt, was insbesondere bei höheren Konzentrationen zu Ausbeuteverlusten durch die Abtrennung zusammen mit dem festen Katalysator führen kann. Deshalb wird Wasser dem Produktgemisch (1) erst nach dem Abtrennen des Katalysators zugegeben. Während der thermischen Behandlung werden die Zwischenprodukte gezielt zum Zielprodukt umgesetzt. Eine solche zweistufige Prozessdurchführung weist große Vorteile gegenüber den aus dem Stand der Technik bekannten Verfahren auf.

Durch diese zweistufige Reaktionsführung kann eine höhere Konzentration an Substanz (II) im Reaktionsgemisch erreicht werden.

Das Verfahren ist für die Herstellung der Substanzen (I) wie z.B. Meropenem (la), Ertapenem (Ib), Imipenem (Ic), Tebipenem (Id), Doripenem (Ie) und den synthetischen Vorstufen von Biapenem (If) und Panipenem (Ig) aus den entsprechenden p-Nitrobenzyl- (PNB) und / oder p-Nitrobenzyloxycarbonyl- (PNZ) geschützen Derivaten (II) geeignet.

Bevorzugt kann dadurch Meropenem (la) aus dem entsprechenden geschützten Meropenemderivat (IIa) hergestellt werden:

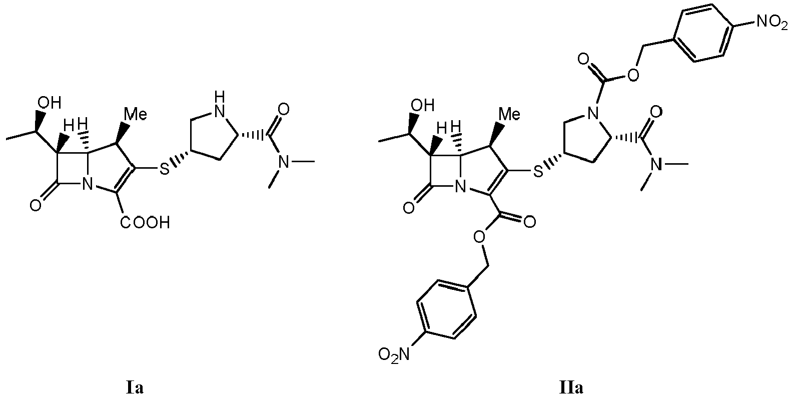

Bevorzugt enthält das erfindungsgemäße Verfahren einen zusätzlichen, nach Schritt d) auszuführenden Schritt, bei dem man ein flüssiges Fällungsmittel zum Produktgemisch (3) hinzufügt und die feste Substanz (I) abtrennt.
Zur Isolierung der festen Substanz (I) können auch andere Methoden wie zum Beispiel eine teilweise Verdampfung des Lösungsmittels in Kombination mit einer Fällung eingesetzt werden.

Das Reaktionsgemisch (1) enthält die Substanz (II), mindestens ein organisches Lösungsmittel und mindestens ein aromatisches Amin. Das Lösungsmittel für die Substanz (II) enthaltende Lösung kann ein organisches Lösungsmittel oder ein Lösungsmittelgemisch dieses Lösungsmittels mit Wasser oder anderen organischen Lösungsmitteln sein. Das organische Lösungsmittel wird bevorzugt aus der Gruppe bestehend aus Tetrahydrofuran (THF), Dioxan, Diethylether, N,N-Dimethylformamid (DMF), Alkoholen, Ethylacetat und Mischungen daraus ausgewählt. Besonders bevorzugt sind THF und dessen Mischungen mit Ethanol oder Methanol. Das Reaktionsgemisch (1) ohne Substanz (II) und Amin besteht bevorzugt zu mehr als 80 Gew.-%, bevorzugt zu wenigstens 90 Gew.-%, besonders bevorzugt zu 95 Gew.-% oder mehr aus einem organischen Lösungsmittel, und zu maximal 20 Gew.-%, bevorzugt zu weniger als 10 Gew.-%, besonders bevorzugt zu 0 bis 5 Gew.-% aus Wasser.
Der Wassergehalt der Lösung sollte möglichst gering sein, um eine möglichst hohe Konzentration der im organischen Lösungsmittel gut löslichen Substanz (II) zu ermöglichen. Daher wird bevorzugt möglichst kein Wasser eingesetzt.
Durch den Restfeuchtegehalt des festen, heterogenen Hydrierkatalysators können im Reaktionsgemisch (1) geringe Mengen an Wasser vorliegen.
Das Reaktionsgemisch (1) enthält bevorzugt von 1 bis 50 Gew.-%, bevorzugt von 3 bis 40 Gew.-%, besonders bevorzugt von 5 bis 30 Gew.-% der Substanz (II).
Als fester heterogener Hydrierkatalysator können alle für diesen Zweck in Frage kommenden Feststoff-Hydrierkatalysatoren ausgewählt werden. Bevorzugt werden solche Hydrierkatalysatoren eingesetzt, die mindestens ein katalytisch aktives Metall enthalten. Bevorzugt ist das katalytisch aktive Metall eines der Gruppen VII B und/oder VIII B des Periodensystems der Elemente, wobei Edelmetalle und Ni bevorzugt sind, insbesondere sind Ru, Rh, Pd, Pt, Re und Ni bevorzugt. Die Metalle können im Hydrierkatalysator als solche und/oder in Form von Oxiden, Hydroxiden, Salzen oder Komplexverbindungen vorliegen. Das katalytisch aktive Metall kann entweder auf einem Träger aufgebracht oder alleine, zum Beispiel als Partikel, eingesetzt werden. Das Trägermaterial ist nicht beschränkt, üblicherweise werden Träger wie Aluminiumoxid, Siliciumdioxid, Eisenoxid, Magnesiumoxid, Zirkoniumdioxid, Titandioxid, Ceroxid, Aktivkohle, Graphit, Ruß oder ähnliche dem Fachmann auf dem Gebiet der Hydrierung bekannte Träger eingesetzt. Der Gehalt an katalytisch aktivem Metall auf dem Träger wird üblicherweise in einem Bereich von 0,1 Gew.-% bis 30 Gew.-% bezogen auf das Gesamtgewicht des Katalysators gewählt. Bevorzugt wird ein Gehalt von 1 Gew.-% bis 15 Gew.-% an katalytisch aktivem Metall auf dem Träger gewählt.
Beispiele für derartige Hydrierkatalysatoren sind Pt/C, Pd/C, Rh/C, Ru/C, Pd/CaCO₃, Pt/Al₂O₃, Pd/Al₂O₃, Ru/Al₂O₃, Rh/Al₂O₃, Pd/Re/C, Pt/Re/C, Pt/V/C, Pt/Fe/C, RuO₂, Raney-Nickel. Bevorzugt wird der Hydrierkatalysator ausgewählt aus der Gruppe bestehend aus Pd/C, Pt/C, Pd/Al₂O₃, Pt/Al₂O₃, Pd/CaCO₃, Ru/Al₂O₃, Pd/Re/C, Pt/Re/C, Pt/V/C, Pt/Fe/C und Raney-Nickel. Besonders bevorzugt sind Pd/C mit 5-10 % Gew.-% Pd und Pt/C mit 5-10 Gew.-% Pt, wobei Pd/C mit 5 Gew.-% Pd (5% Pd/C) und Pt/C mit 5 Gew.-% Pt (5% Pt/C) ganz besonders bevorzugt sind.

Die Menge des festen heterogenen Hydrierkatalysators kann vom Fachmann frei gewählt werden, wobei das Molverhältnis von katalytisch aktivem Metall des Hydrierkatalysators zu Substanz (II) üblicherweise in einem Bereich von 1:1 bis 1:10.000 liegt. Weiter bevorzugt ist ein Bereich von 1:10 bis 1:1.000, besonders bevorzugt ist ein Bereich von 1:20 bis 1:300.

Der feste heterogene Hydrierkatalysator kann mit einem Restfeuchtegehalt eingesetzt werden, wobei der Restfeuchtegehalt über den Trocknungsverlust angegeben wird. Der Trocknungsverlust kann im Bereich von 0 Gew.-% bis 80 Gew.-% liegen, wobei ein Trocknungsverlust im Bereich von 0 Gew.-% bis 65 Gew.-% bevorzugt ist.

Das aromatische Amin wird bevorzugt aus der Gruppe bestehend aus Anilin, substituierten Anilinen (Aminobenzolderivaten), polyaromatischen Aminen, heteroaromatischen Aminen (wie z.B. Aminopyridine, - thiophene, -furane etc.), die weiter substituiert sein können, gewählt. Besonders bevorzugt werden Aniline wie Anilin, 4-Ethylanilin, p-Aminotoluol, m-Aminotoluol, o-Aminotoluol eingesetzt, besonders bevorzugt wird p-Aminotoluol eingesetzt.
Das Molverhältnis [das zum Reaktionsgemisch (1) zugegebene aromatische Amin] / [Substanz (II)] ist bevorzugt von 0,1 bis 20, bevorzugt von 0,5 bis 10, besonders bevorzugt von 1 bis 7.

Der Wasserstoffdruck ist für die Hydrierung nicht essentiell, wobei durch einen erhöhten Druck die Reaktionszeit verkürzt werden kann. Der absolute Reaktionsdruck wird üblicherweise mit einer Wasserstoffquelle in einem Bereich von 1 - 200 bar, vorzugsweise von 1,1 - 100 bar, und besonders bevorzugt von 2 - 20 bar, eingestellt und konstant gehalten. Für die Zwecke der Umsetzung kann Wasserstoffgas oder eine Mischung aus Wasserstoffgas mit Inertgas, z.B. handelsübliches Formiergas der Zusammensetzung 5 % H₂/95 % N₂, eingesetzt werden. Die bevorzugte Wasserstoffquelle ist Wasserstoffgas.

Die Temperatur während der Umsetzung mit Wasserstoff kann vom Fachmann in Abhängigkeit vom Lösungsmittel und der thermischen Stabilität des Carbapenemderivats frei gewählt werden. Üblicherweise wird eine Temperatur im Bereich von 0 °C bis zum Siedepunkt des gewählten Lösungsmittels eingestellt. Die Umsetzung mit Wasserstoff wird vorzugsweise bei einer Temperatur im Bereich von 0 - 60 °C durchgeführt, weiter bevorzugt ist eine Temperatur im Bereich von 15 - 50 °C, besonders bevorzugt ist eine Temperatur im Bereich von 15 - 25°C. Die Umsetzung ist üblicherweise beendet, wenn keine H₂-Aufnahme mehr stattfindet. Die H₂-Aufnahme kann während der Umsetzung mit üblichen Methoden überwacht werden.

Der feste heterogene Hydrierkatalysator kann mit Hilfe jeder dem Fachmann bekannten Methode zur Abtrennung von Feststoffen aus Flüssigkeiten aus dem Produktgemisch (1) abgetrennt werden. Geeignete Methoden sind z.B. Filtration, Zentrifugation, Sedimentation oder Ultraschallseparation.

Wasser wird zum Produktgemisch (2) nach Hydrierung und Abtrennung des Katalysators im Schritt c) zugegeben. Dabei ist das Gewichtsverhältnis von zugegebenem Wasser zum Produktgemisch (2) von 0,1 bis 10, bevorzugt von 0,3 bis 7, besonders bevorzugt von 0,5 bis 5.

Die Temperatur während der thermischen Behandlung mit Wasser im Schritt d) wird auf Werte von 0 - 100 °C, bevorzugt 15-60 °C, besonders bevorzugt 25 - 50 °C eingestellt und besonders bevorzugt wird die Umsetzung bei 40 °C durchgeführt. Gegebenenfalls wird während der Umsetzung gerührt. Thermische Behandlung im Schritt d) wird innerhalb von 0,1 bis 10 Stunden, bevorzugt von 0,3 bis 5 Stunden, besonders bevorzugt innerhalb von 0,5 bis 1,5 Stunden durchgeführt.

Das Fällungsmittel dient dazu, die Löslichkeit der Substanz (I) im Produktgemisch (3) herabzusetzen. Bevorzugt wird ein mindestens teilweise mit Wasser mischbares Lösungsmittel verwendet. Besonders bevorzugt wird ein aprotisches Lösungsmittel als flüssiges Fällungsmittel eingesetzt. Das Fällungsmittel wird besonders bevorzugt aus der Gruppe bestehend aus Aceton und THF ausgewählt.

Um eine möglichst komplette Abtrennung der Substanz (I) aus dem Produktgemisch (3) zu erreichen, wird das Produktgemisch (3) nach der thermischen Behandlung im Schritt d) und vor Zugabe eines Fällungsmittels bevorzugt abgekühlt. Die Temperatur während der Fällung beträgt bevorzugt -30 bis 30°C, besonders bevorzugt -10 bis +15°C. Methoden zur Kühlung sind dem Fachmann bekannt.

Die Substanz (I) kann mit Hilfe jeder dem Fachmann bekannten Methode zur Abtrennung von Feststoffen aus Flüssigkeiten aus der Produktmischung abgetrennt werden. Geeignete Methoden sind Filtration, Zentrifugation oder Sedimentation.

Das isolierte Rohprodukt, das die Substanz (I) enthält, kann durch übliche Methoden gereinigt und so in der gewünschten Reinheit erhalten werden. Die Reinigung kann dabei mindestens einen dem Fachmann gebräuchlichen Schritt wie Waschen, Extrahieren, Umkristallisation, Filtration, oder Trocknen umfassen.

### BEISPIELE

### Einsatzstoff

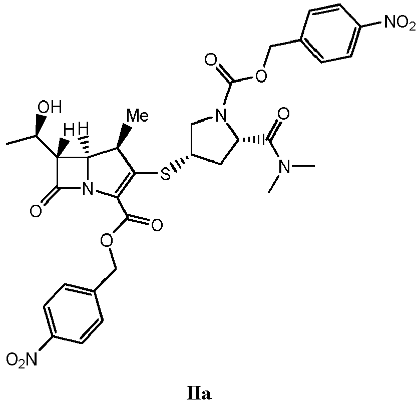

### Analytik

Zur Bestimmung des Trocknungsverlustes wurden die Katalysatoren in einem Vakuumtrockenschrank bei 80 °C bis zur Massenkonstanz getrocknet (Trocknungsverlust (%) = (Masse vor Trocknung - Masse nach der Trocknung)*100 %/(Masse vor der Trocknung).

Der Gehalt an reinem Zielprodukt Meropenem im isolierten und getrockneten Niederschlag (Rohprodukt) wurde mittels HPLC (HPLC System 1100 Serie mit einem DAD, Fa. Agilent, und einem MultiPurpose Sampler 3, Fa. Gerstel) analog der Vorschrift nach USP 38 (United States Pharmakopoeia) gegen Meropenem-Trihydrat (USP Reference Standard) als externen Standard ermittelt. Alle angegebenen Ausbeuten beziehen sich somit auf die isolierten Ausbeuten an Zielprodukt Meropenem bestimmt durch die Reinheit des isolierten Rohprodukts.

### 1. Erfindungsgemäße Beispiele

### Allgemeine Versuchsvorschrift (Beispiele 1-14)

In einen Druckreaktor werden die Lösung, der Katalysator und der Hilfsstoff eingewogen. Der Reaktor wird verschlossen, mit Stickstoff inertisiert und auf die Reaktionstemperatur gebracht. Der Reaktor wird mit Wasserstoff befüllt, der Druck im Reaktor wird mit Wasserstoff auf den Reaktionsdruck eingestellt und der Rührer wird gestartet. Die Reaktion wird beendet, wenn keine H₂-Aufnahme mehr beobachtet wird. Nach Inertisierung mit Stickstoff wird der Reaktor geöffnet. Der Katalysator wird mit einem Filter von der Reaktionsmischung abgetrennt.

Zur Aufarbeitung und Ausbeutebestimmung wird ein Teil des erhaltenen Filtrats verwendet. Zu der filtrierten Reaktionsmischung wird unter Rühren entionisiertes Wasser zugegeben, die Mischung wird für 1 h bei 40 °C gerührt. Danach wird sie im Eisbad auf 0-5 °C abgekühlt, nach Zugabe von Aceton wird die Mischung 1 Stunde lang im Eisbad gerührt, um das Reaktionsprodukt zu fällen. Der gefällte Niederschlag wird abfiltriert und bei 35 °C im Vakuumtrockenschrank getrocknet. Die Ermittlung der Ausbeute erfolgt nach Gehaltsbestimmung des isolierten und getrockneten Niederschlages mittels HPLC analog der Vorschrift nach USP 38 gegen Meropenem-Trihydrat (USP Reference Standard) als externen Standard.

### 2. Vergleichsbeispiele

*VB 1* - analog zu WO2007/104221 (einstufige Umsetzung)
   1,4 g Verbindung (IIa) werden in 35 ml THF aufgelöst, 28 ml Wasser werden unter Rühren dazugegeben. In einen Druckreaktor werden 6,0 g dieser Lösung, 2,6-Lutidin und Katalysator (10 % Pd/C von Evonik Industries) eingewogen. Der Reaktor wird verschlossen, mit Stickstoff inertisiert und auf die Reaktionstemperatur gebracht. Der Reaktor wird mit Wasserstoff befüllt, der Druck im Reaktor wird mit Wasserstoff auf 18 barü eingestellt und der Rührer wird gestartet. Die Reaktion wird nach einer Stunde beendet. Nach Inertisierung mit Stickstoff wird der Reaktor geöffnet. Der Katalysator wird mit einem Filter von der Reaktionsmischung abgetrennt.
   Zur Produktfällung und Ausbeutebestimmung wird ein Teil (3,0 g) des erhaltenen Filtrats verwendet. Zu der filtrierten Reaktionsmischung werden 5,5 ml Aceton zugegeben. Produktfällung erfolgt unter Kühlung im Eisbad bei ca. 5 °C. Es werden nach 30 min weitere 2,7 ml Aceton tropfenweise bei gleicher Temperatur zugegeben. Nach 30 min wird der gefällte Niederschlag abfiltriert und bei 40 °C im Vakuumtrockenschrank getrocknet. Die Ermittlung der Ausbeute erfolgt nach Konzentrationsbestimmung der wässrigen Lösung des getrockneten Niederschlages mittels HPLC analog der Vorschrift nach USP 38 gegen Meropenem-Trihydrat (USP Reference Standard) als externen Standard.
VB 2: Der Versuch wird analog zu VB 1 durchgeführt, allerdings wird als Katalysator Pd/C mit 5 Gew.-% Pd eingesetzt.
VB 3: Der Versuch wird nach der allgemeinen Versuchsbeschreibung für die erfindungsgemäßen Beispiele durchgeführt, allerdings wird 2,6-Lutidin statt p-Aminotoluol verwendet.
VB 4: Der Versuch wird nach der allgemeinen Versuchsbeschreibung für die erfindungsgemäßen Beispiele durchgeführt, allerdings wird n-Butylamin verwendet.
VB 5: Der Versuch wird nach der allgemeinen Versuchsbeschreibung für die erfindungsgemäßen Beispiele durchgeführt, allerdings wird Ethylendiamin verwendet.
VB 6: Der Versuch wird nach der allgemeinen Versuchsbeschreibung für die erfindungsgemäßen Beispiele durchgeführt, allerdings wird kein Amin eingesetzt.
VB 7: Der Versuch wird nach der allgemeinen Versuchsbeschreibung für die erfindungsgemäßen Beispiele durchgeführt, allerdings wird kein Amin eingesetzt.

Die Einsatzstoffe und Reaktionsbedingungen können Tabelle 2 entnommen werden.

**Tabelle 1: Einsatzstoffe und Reaktionsbedingungen - Erfindungsgemäße Beispiele**

| **Beispiel** | **Lösung IIa in THF** | | **Aromatisches Amin** | **Hydrier ungszei t^{[f]}** | **Amin / IIa^{[k]}** | **Katalys ator** | **Produktg emisch (2)** | **Wasser^{[d]}** | **Wasser/ Produktge misch (2)^{[j]}** | **Aceton** | **Ausbe ute** | **LöMi/Iₐ^{[l]}** |
|---|---|---|---|---|---|---|---|---|---|---|---|---|
| | g | Gew.-% **IIa** | mmol | min | mol/mol | mg | g | g | g/g | ml | % | g/mmol |
| 1 | 5,38 | 7,25 | p-Aminotoluol 0,8 | 36 | 1,4 | 31**^{[a]}** | 2,74 | 3,0 | 1,1 | 8,0**^{[e]}** | 79 | 40 |
| 2 | 5,56 | 11,3 | p-Aminotoluol 1,0 | 30 | 1,1 | 51**^{[a]}** | 2,76 | 5,0 | 1,8 | 13,5**^{[e]}** | 79 | 38 |
| 3 | 5,67 | 17,0 | p-Aminotoluol 1,6 | 22 | 0,7 | 50,3**^{[a]}** | 2,71 | 8,0 | 3,0 | 21,5**^{[e]}** | 70 | 43 |
| 4 | 5,61 | 18,4 | p-Aminotoluol 1,6 | 41 | 1,1 | 80,6**^{[a]}** | 2,91 | 8,0 | 2,7 | 21,5**^{[e]}** | 80 | 32 |
| 5 | 5,61 | 18,4 | p-Aminotoluol 3,2 | 41 | 2,2 | 82**^{[a]}** | 2,85 | 8,0 | 2,8 | 21,5**^{[e]}** | 77 | 35 |
| 6 | 5,62 | 18,4 | p-Aminotoluol 4,0 | 41 | 2,7 | 82**^{[a]}** | 2,84 | 8,0 | 2,8 | 21,5**^{[e]}** | 84 | 33 |
| 7 | 5,85 | 18,4 | o-Aminotoluol 1,6 | 35 | 1,0 | 81**^{[a]}** | 2,79 | 8,0 | 2,9 | 21,5**^{[e]}** | 80 | 34 |
| 8 | 5,70 | 18,4 | m-Aminotoluol 1,6 | 35 | 1,1 | 80**^{[a]}** | 2,80 | 8,0 | 2,9 | 21,5**^{[e]}** | 77 | 35 |
| 9 | 5,65 | 18,4 | 4-Ethylanilin 1,6 | 40 | 1,1 | 80**^{[a]}** | 2,70 | 8,0 | 3,0 | 21,5**^{[e]}** | 75 | 37 |
| 10 | 5,58 | 18,4 | Anilin 1,6 | 40 | 1,1 | 81**^{[a]}** | 2,74 | 8,0 | 2,9 | 21,5**^{[e]}** | 73 | 37 |
| 11 | 5,43 | 16,2^{[g]} | p-Aminotoluol 1,6 | 27 | 1,3 | 86^{[a]} | 2,73 | 8,0 | 2,9 | 21,5**^{[e]}** | 82 | 38 |
| 12 | 5,77 | 25,0^{[g]} | p-Aminotoluol 2,5 | 53 | 1,2 | 80^{[a]} | 2,86 | 8,0 | 2,8 | 21,5^{[e]} | 69 | 28 |
| 13 | 5,64 | 18,6 | p-Aminotoluol 1,6 | 90 | 1,1 | 80^{[b]} | 2,68 | 8,0 | 3,0 | 21,5**^{[e]}** | 76 | 36 |
| 14 | 5,54 | 19,0^{[g]} | p-Aminotoluol 1,6 | 92 | 1,1 | 81^{[b]} | 2,76 | 8,0 | 2,9 | 21,5**^{[e]}** | 77 | 34 |

| | | | | | | | | | | | | |
|---|---|---|---|---|---|---|---|---|---|---|---|---|
| [a] Pt/C mit 5 Gew.-% Pt, Trocknungsverlust = 66,0 %, die Katalysatoreinwaage bezieht sich jeweils auf die Katalysatortrockensubstanz, der Katalysator wird vor Benutzung nicht getrocknet; [b] Pd/C mit 5 Gew.-% Pd, Trocknungsverlust = 56,0 %, die Katalysatoreinwaage bezieht sich jeweils auf die Katalysatortrockensubstanz, der Katalysator wird vor Benutzung nicht getrocknet; [c] Pd/C mit 10 Gew.-% Pd, Trocknungsverlust = 56,5 %, die Katalysatoreinwaage bezieht sich jeweils auf die Katalysatortrockensubstanz, der Katalysator wird vor Benutzung nicht getrocknet; [d] Rühren für 1h bei 40°C; [e] Rühren für 1 h im Eisbad; [f] 25 °C, 18 barü H₂ [g] in THF/EtOH (Volumenverhältnis THF : EtOH =2:1 [h] in THF/H₂O (Volumenverhältnis THF:Wasser = 1,25:1) entspricht 4,50 % bezogen nur auf THF) [i] in THF/EtOH (Volumenverhältnis THF : EtOH = 2:1) [j] Gewichtsverhältnis [k] Molarverhältnis [l] Gesamtmasse aller organischen Lösungsmittel in g (Lösungsmittel in Reaktionsgemisch (1) + Fällungsmittel) bezogen auf Molmenge (mmol) von gebildetem Produkt | | | | | | | | | | | | |

**Tabelle 2: Einsatzstoffe und Reaktionsbedingungen - Vergleichsbeispiele**

| **Beispiel** | **Lösung IIa in THF** | | **Base bzw. Amin** | **Hydrierungszeit^{[f]}** | **Base bzw. Amin /IIa^{[k]}** | **Katalysator** | **Produktgemisch (2)** | **Wasser^{[d]}** | **Wasser/ Produktgemisch (2)^{[j]}** | **Aceton** | **Ausbeute** | **LöMi/Ia^{[l]}** |
|---|---|---|---|---|---|---|---|---|---|---|---|---|
| | g | Gew.-% **IIa** | mmol | min | mol/mol | mg | g | ml | ml | ml | % | g/mmol |
| VB 1 | 6,00 | 2,37^{[h]} | 2,6-Lutidin 0,70 | 60 | 7,9 | 12^{[c]} | 3,00 | - | 0,0 | 8,2 | 52 | 179 |
| VB 2 | 6,00 | 2,37^{[h]} | 2,6-Lutidin 0,70 | 60 | 7,9 | 24^{[b]} | 3,00 | - | 0,0 | 8,2 | 49 | 190 |
| VB 3 | 5,58 | 18,2 | 2,6-Lutidin 1,6 | 35 | 1,1 | 81^{[a]} | 2,77 | 8,0 | 2,9 | 21,5**^{[e]}** | 37 | 74 |
| VB 4 | 5,61 | 18,2 | n-Butylamin 1,6 | 40 | 1,1 | 80^{[a]} | 2,80 | 8,0 | 2,9 | 21,5**^{[e]}** | 0,59 | 4600 |
| VB 5 | 5,58 | 18,2 | Ethylendiamin 1,6 | 35 | 1,1 | 80^{[a]} | 2,79 | 8,0 | 2,9 | 21,5**^{[e]}** | 0 | - |
| VB 6 | 5,45 | 7,12 | - | 90 | 2,9 | 30^{[a]} | 2,76 | 2,0 | 0,7 | 21,5^{[e]} | 60 | 116 |
| VB 7 | 5,47 | 16,3^{[i]} | - | 27 | 1,3 | 81^{[a]} | 2,80 | 8,0 | 2,9 | 21,5^{[e]} | 22 | 135 |

| | | | | | | | | | | | | |
|---|---|---|---|---|---|---|---|---|---|---|---|---|
| [a] Pt/C mit 5 Gew.-% Pt, Trocknungsverlust = 66,0%, die Katalysatoreinwaage bezieht sich jeweils auf die Katalysatortrockensubstanz, der Katalysator wird vor Benutzung nicht getrocknet; [b] Pd/C mit 5 Gew.-% Pd, Trocknungsverlust = 56,0%, die Katalysatoreinwaage bezieht sich jeweils auf die Katalysatortrockensubstanz, der Katalysator wird vor Benutzung nicht getrocknet; [c] Pd/C mit 10 Gew.-% Pd, Trocknungsverlust = 56,5%, die Katalysatoreinwaage bezieht sich jeweils auf die Katalysatortrockensubstanz, der Katalysator wird vor Benutzung nicht getrocknet; [d] Rühren für 1h bei 40°C; [e] Rühren für 1h im Eisbad; [f] 25°C, 18 barü H₂ [g] in THF/EtOH (Volumenverhältnis THF : EtOH =2:1 [h] in THF/H₂O (Volumenverhältnis THF:Wasser = 1,25:1) entspricht 4,50 % bezogen nur auf THF) [i] in THF/EtOH (Volumenverhältnis THF : EtOH = 2:1) [j] Gewichtsverhältnis [k] Molarverhältnis [l] Gesamtmasse aller organischen Lösungsmittel in g (Lösungsmittel in Reaktionsgemisch (1) + Fällungsmittel) bezogen auf Molmenge (mmol) von gebildetem Produkt | | | | | | | | | | | | |

## Patentansprüche

1. Verfahren zur Herstellung einer Substanz der Formel (I) aus einer Substanz der Formel (II): 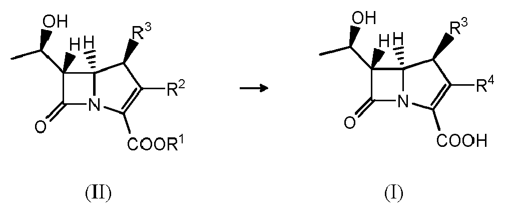 R¹ = p-Nitrobenzyl 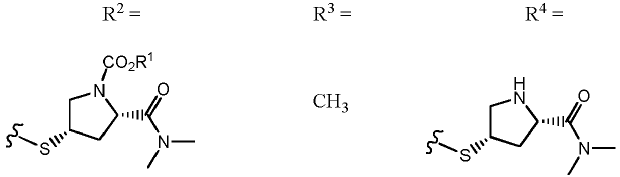 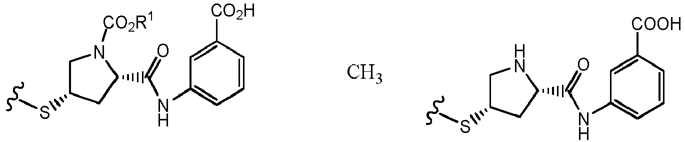 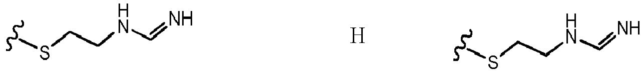 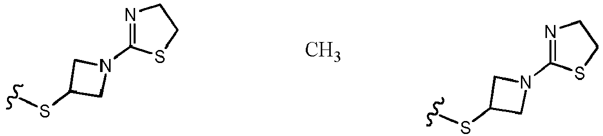 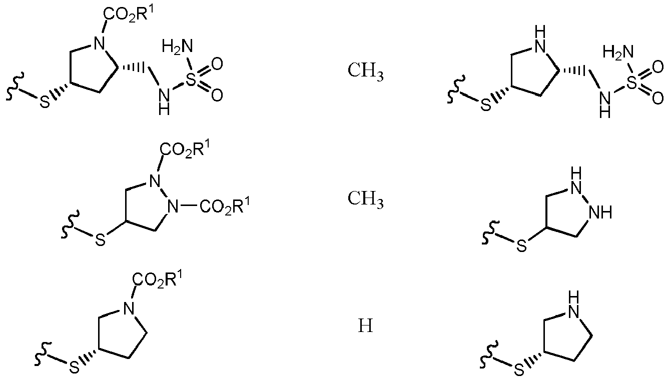 enthaltend die Schritte:
a) Hydrierung mit Wasserstoff eines Reaktionsgemisches (1) enthaltend Substanz (II), mindestens ein organisches Lösungsmittel und mindestens ein aromatisches Amin in Gegenwart eines heterogenen Hydrierkatalysators unter Bildung eines Produktgemisches (1),
b) Abtrennung des heterogenen Katalysators aus dem Produktgemisch (1) unter Bildung eines Produktgemisches (2),
c) Hinzufügen von Wasser zum Produktgemisch (2), wobei das Gewichtsverhältnis von zugegebenem Wasser zum Produktgemisch (2) von 0,1 bis 10 ist,
d) Thermische Behandlung des Produktgemisches (2) unter Bildung eines Produktgemisches (3) enthaltend Substanz (I).

2. Verfahren nach Anspruch 1, **dadurch gekennzeichnet, dass** Substanz (II) 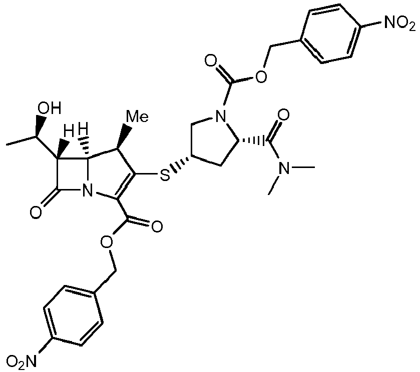 ist und Substanz (I) Meropenem ist.

3. Verfahren nach den Ansprüchen 1 oder 2, **dadurch gekennzeichnet, dass** nach dem Schritt d) Hinzufügen eines flüssigen Fällungsmittels zum Produktgemisch (3) und Abtrennung der festen Substanz (I) durchgeführt wird.

4. Verfahren nach den Ansprüchen 1 bis 3, **dadurch gekennzeichnet, dass** das organische Lösungsmittel aus der Gruppe bestehend aus Tetrahydrofuran, Dioxan, Diethylether, N,N-Dimethylformamid, Ethylacetat, Methanol, Ethanol, Propanol und Gemischen davon ausgewählt ist.

5. Verfahren nach den Ansprüchen 1 bis 4, **dadurch gekennzeichnet, dass** die Hydrierung bei einem Reaktionsdruck von 1 bar bis 200 bar durchgeführt wird.

6. Verfahren nach den Ansprüchen 1 bis 5, **dadurch gekennzeichnet, dass** der heterogene Hydrierkatalysator ein Pd/C Katalysator mit 1-15 Gew.-% Pd oder ein Pt/C Katalysator mit 1-15 Gew.-% Pt ist.

7. Verfahren nach den Ansprüchen 1 bis 6, **dadurch gekennzeichnet, dass** der heterogene Hydrierkatalysator in einem Mol-Verhältnis von katalytisch aktivem Metall des Katalysators zu Substanz (II) von 1:1 bis 1:10.000 eingesetzt wird.

8. Verfahren nach den Ansprüchen 1 bis 7, **dadurch gekennzeichnet, dass** das aromatische Amin aus der Gruppe bestehend aus Anilinen, polyaromatischen Aminen und heteroaromatischen Aminen ausgewählt ist.

9. Verfahren nach den Ansprüchen 1 bis 8, **dadurch gekennzeichnet, dass** das molare Verhältnis des zugegebenen aromatischen Amins zur Substanz (II) von 0,1 bis 20 ist.

10. Verfahren nach den Ansprüchen 3 bis 9, **dadurch gekennzeichnet, dass** das Fällungsmittel ein mindestens teilweise mit Wasser mischbares aprotisches Lösungsmittel ist.

11. Verfahren nach den Ansprüchen 1 bis 10, **dadurch gekennzeichnet, dass** das Reaktionsgemisch (1) ohne Substanz (II) und Amin zu 0 bis 20 Gew.- % Wasser besteht.

12. Verfahren nach den Ansprüchen 1 bis 11, **dadurch gekennzeichnet, dass** die thermische Behandlung des Produktgemisches (2) bei Temperaturen von 0 bis 100 °C stattfindet.

13. Verfahren nach den Ansprüchen 1 bis 12, **dadurch gekennzeichnet, dass** die thermische Behandlung des Produktgemisches (2) innerhalb von 0,1-10 Stunden stattfindet.

14. Verfahren nach den Ansprüchen 1 bis 13, **dadurch gekennzeichnet, dass** das Produktgemisch (3) nach dem Schritt d) auf -10 bis +15 °C abgekühlt wird.

15. Verfahren nach den Ansprüchen 1 bis 14, **dadurch gekennzeichnet, dass** das Reaktionsgemisch (1) 5 bis 50 Gew.-% der Substanz (II) enthält.
